# EUROPEAN PATENT APPLICATION

(11) **EP 4 393 534 A1**
(43) Date of publication of application: **03.07.2024**
(21) Application number: 21958156.8
(22) Date of filing: 08.11.2021
(51) Int. Cl.: A61M 25/00

(54) **MEDICAL TUBE**

(30) Priority: 26.09.2021 CN 202122344424 U
(71) Applicant: Hydro Caresys (Huizhou) Medical Co. Ltd, Guangdong 516081 (CN)
(72) Inventor: SUN, Guangyu, Guangdong 516081 (CN); WU, Yan, Guangdong 516081 (CN)
(74) Representative: Zaboliene, Reda
(86) International application number: PCT/CN2021/129262
(87) International publication number: WO 2023/045043

(57) **Abstract**

A medical tubing comprises: an inner tube (10), wherein the inner tube (10) is a flexible tube, and a ratio of the wall thickness of the inner tube (10) to the inner diameter of the inner tube (10) ranges from 0.1 to 10; and one or more braid layers (20) that are successively overlapped from inside to outside, the braid layers (20) is disposed on the outer periphery of the inner tube (10), each of the braid layers (20) comprises one or more flexible braided wires (21) that are overlapped in an interlaced manner, and the wire diameter of the flexible braided wires (21) is 0.01-10 mm. The medical tubing can ensure the pressure performance of the tubing and can improve the flexibility and bending resistance of the tubing.

## Description

### TECHNICAL FIELD

The disclosure relates to the technical field of medical devices, more particularly to a medical tubing.

### BACKGROUND

Existing braided medical tubing usually has shortcomings such as poor pressure resistance, low bending resistance, and low flexibility, and is not convenient to use. In the modern medical technology, in particular the catheter for introduction of fluids, requires high pressure resistance, and certain flexibility and bending resistance as well.

### SUMMARY OF THE UTILITY MODEL

One of the goals of the disclosure is to provide a medical tubing having improved flexibility, high bending resistance, and increased pressure resistance.

The above goal of the disclosure is achieved by a technical solution as follows.

A medical tubing comprises:
an inner tube, wherein the inner tube is a flexible tube, and a ratio of a wall thickness of the inner tube to an inner diameter of the inner tube ranges from 0.1 to 10;
one braided layer or more braided layers that are successively overlapped from inside to outside, wherein the braided layer is disposed on the outer periphery of the inner tube, the braided layer comprises one flexible braided wire or more flexible braided wires that are overlapped in an interlaced manner, and the flexible braided wire has a wire diameter of 0.01-10mm.

In some implementations of the disclosure, the medical tubing may further comprise:
a cladding layer disposed outer than the braided layer, wherein the cladding layer may be made of PVC, polyurethane, pp, nylon, rubber, or silica gel.

In some implementations of the disclosure, the braided layer may further comprise at least two flexible braided wires overlapped in an interlaced grid-like manner.

In some implementations of the disclosure, each of the flexible braided wires and an axial direction of the inner tube are at an angle of 1°-89°.

In some implementations of the disclosure, each of the flexible braided wires and an axial direction of the inner tube are at an angle of 40°-50°.

In some implementations of the disclosure, two groups of flexible braided wires are provided, which respectively extend towards two axially opposite ends of the inner tube.

In some implementations of the disclosure, the wire diameter of the flexible braided wires may be 0.5-1.5mm.

In some implementations of the disclosure, the ratio of the wall thickness of the inner tube to the inner diameter of the inner tube may range from 0.5 to 2.

In some implementations of the disclosure, the wall thickness of the inner tube may be 0.5mm-1.5mm, and the inner diameter of the inner tube may be 1mm-1.5mm.

In some implementations of the disclosure, the inner tube may be made of PVC, polyurethane, pp, nylon, rubber, or silica gel.

In some implementations of the disclosure, the flexible braided wires may be made of stainless steel wire, nylon wire, or KEVLAR^{®}.

As the medical tubing of the disclosure comprises the inner tube which is the flexible tube and the braided layer disposed on the outer periphery of the inner tube and weaved by the flexible braided wires, it ensures the flexibility and bending resistance of the whole tube. In addition, due to the inner tube with appropriate wall thickness and inner diameter and the braided layer weaved by flexible braided wires with an appropriate wire diameter, the maximum mechanical strength of the tube can be effectively increased.

### BRIEF DESCRIPTION OF THE DRAWINGS

The disclosure will be further explained below in detail with reference to figures and preferred embodiments. It should be understood by those skilled in the art that the drawings are illustrative of the preferred embodiments and are not intended to limit the present disclosure. In addition, unless specified otherwise, the drawings are intended to conceptually represent compositions or configurations of the described object. The drawings are not necessarily to scale and may include exaggerated illustration.
FIG.1 is a schematic structural view of a medical tubing according to some embodiment of the disclosure;
FIG.2 is a schematic structural view of a medical tubing according to some embodiment of the disclosure.
In the drawings: 10. inner tube; 20. braided layer; 21. flexible braided wires; 30. cladding layer.

### DETAILED DESCRIPTION OF ILLUSTRATED EMBODIMENTS

The preferred embodiments of the disclosure will be described below in detail in conjunction with the drawings. It should be appreciated by those skilled in the art that the description is illustrative and exemplary and are not intended to limit the scope.

Firstly, it should be noted that the terms, such as "top", "bottom", "upward", and "downward" as used in the description refer to position and orientation relationships as shown in the drawings. They are relative terms and may change depending on different situations or different states, and should not be regarded as a limitation to the present disclosure.

It should be noted that the term "comprise" does not exclude other elements or steps, and "a" or "an" does not exclude plural.

In addition, it should be noted that each of the technical features described or implied in the embodiments, or each of the technical features illustrated or implied in the drawings, or equivalents thereof, can be combined in any form, to obtain other embodiments without departing from the present disclosure.

It should be understood that the terms such as "first" and "second" as used in the description are used to describe various information and are not intended to make any limitation for the various information. These terms are used to distinguish the various information. For example, "first" information may be referred to as "second" information, and similarly "second" information may be referred to as "first" information, without departing from the present disclosure.

It should be noted that the same reference signs in different drawings indicate same or similar parts.

### Embodiment 1

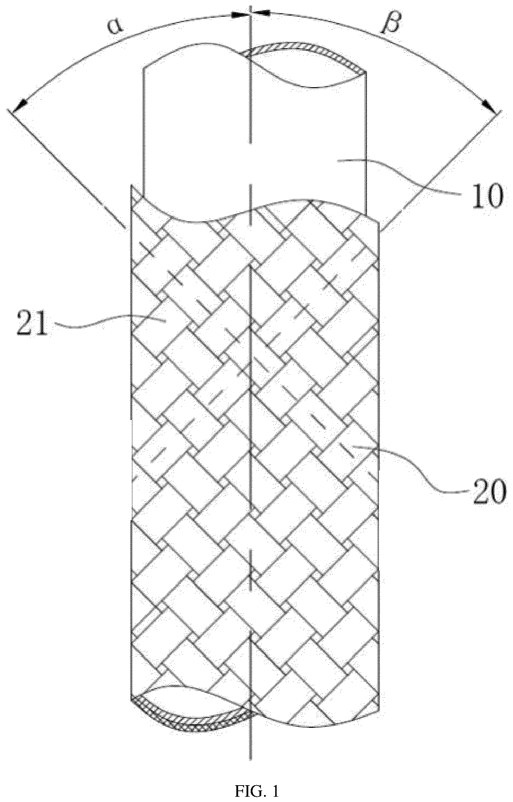

Referring to FIG.1, a medical tubing according to an embodiment of the disclosure comprises: an inner tube 10 and a braided layer 20 disposed on the outer periphery of the inner tube 10. Herein, the inner tube 10 is a flexible tube, and the ratio of the wall thickness of the inner tube 10 to the inner diameter of the inner tube 10 ranges from 0.1 to 10. One braided layer 20 may be provided, or more braided layers 20 that are successively overlapped from inside to outside may be provided. The braided layer 20 comprises one flexible braided wire 21 or more flexible braided wires 21 that are overlapped in an interlaced manner. The flexible braided wires 21 have a wire diameter of 0.01-10mm.

As the inner tube 10 of the medical tubing in the embodiment uses the flexible tube and the braided layer 20 is weaved by the flexible braided wires 21, the flexibility and bending resistance of the whole medical tubing can be ensured. Furthermore, as the medical tubing uses the combination of the inner tube 10 with appropriate wall thickness and inner diameter and the braided layer 20 weaved by flexible braided wires 21 with an appropriate wire diameter, the performance of the tube in terms of withstanding the liquid pressure can be effectively increased. In the embodiment, the medical tubing can withstand the pressure in a range of 0.01MPa-500Mpa.

Furthermore, in some implementations of the disclosure, the braided layer 20 may comprise at least two flexible braided wires 21 overlapped in an interlaced grid-like manner. See FIG.1 for more details. Such weaving manner can ensure good pressure resistance.

Referring to FIG.1, two groups of flexible braided wires 21 are provided in the embodiment. Herein, one group of flexible braided wires 21 extend at a weaving angle of α relative to the axial direction of the inner tube 10, and another group of flexible braided wires 21 extend at a weaving angle of β relative to the axial direction of the inner tube 10. In addition, α and β may be the same, or different. Exemplarily, both α and β may be 1°-89°. The direction of the applied force can be controlled depending on the weaving angle, whereby the pressure strength can be improved.

Preferably, in order to improve the pressure strength, both α and β may be preferably 40°-50°, that is, the two groups of flexible braided wires 21 are at an angle of 80°-100°.

In addition, in case that more braided layers 20 may be provided, the angles α and β of each of the braided layers 20 may be the same or different, which is not limited herein.

Exemplarily, in the embodiment, the wire diameter of the flexible braided wires 21 may be 0.5-1.5mm.

In some implementations of the disclosure, the ratio of the wall thickness of the inner tube 10 to the inner diameter of the inner tube 10 may range from 0.5 to 2. By increasing the wall thickness of the inner tube 10, the pressure strength of the tube can be effectively improved. Alternatively, the inner diameter of the inner tube 10 may be 1mm-1.5mm, and the wall thickness of the inner tube 10 may be 0.5mm-1.5mm.

In order to ensure the pressure performance, the inner tube 10 may be made of a high-strength soft metal or non-metallic material. Exemplarily, the inner tube 10 may be made of any one of PVC, polyurethane, pp, nylon, rubber, and silica gel.

In addition, in order to ensure high resistance to bending and optimized pressure performance, the braided wires may be made of a soft metal or non-metallic material. Exemplarily, in some implementations of the disclosure, the flexible braided wires 21 may be made of any one of stainless steel wire, nylon wire, and KEVLAR^{®}.

In conclusion, as the medical tubing according to the embodiment of the disclosure comprises the inner tube 10 which is the flexible tube and the braided layer 20 disposed on the outer periphery of the inner tube 10 and weaved by the flexible braided wires 21, it ensures the flexibility and bending resistance of the whole tube. In addition, due to the inner tube 10 with appropriate wall thickness and inner diameter and the braided layer 20 weaved by flexible braided wires 21 with an appropriate wire diameter, the maximum mechanical strength of the tube can be effectively increased.

### Embodiment 2

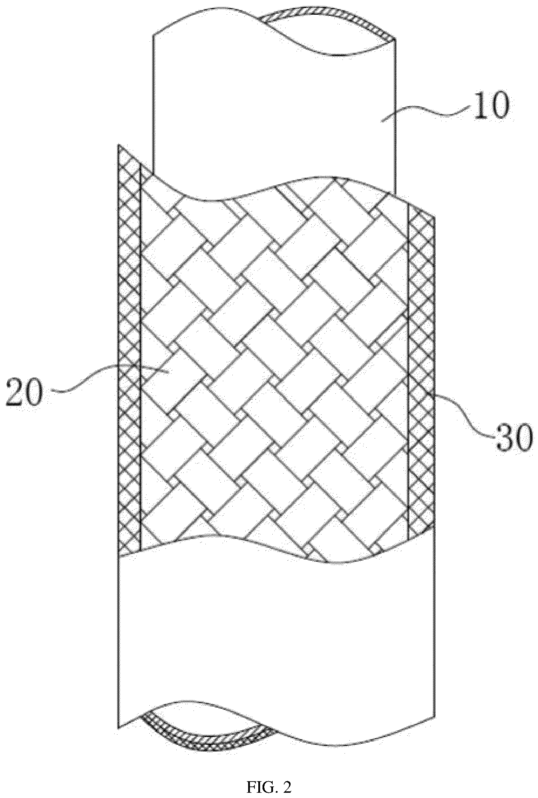

Referring to FIG.2, a medical tubing according to an embodiment of the disclosure merely differs from Embodiment 1 in that the medical tubing in the present embodiment further comprises a cladding layer 30 disposed outer than the braided layer 20, and the cladding layer 30 may be made of any one of PVC, polyurethane, pp, nylon, rubber, and silica gel.

The disclosure is described in the description in conjunction with the drawings, to enable those skilled in the art to practice the subject matter of the disclosure, including by manufacturing or using any device or system, using appropriate materials or using any method for combination. The scope of the disclosure is defined by technical solutions of the appended claims, and covers all equivalent embodiments obtained by those skilled in the art and falling in the scope of the technical solutions of the appended claims, which may have literally difference but be substantially the same.

## Claims

1. A medical tubing, **characterized by** comprising:
an inner tube (10), the inner tube (10) being a flexible tube, and a ratio of a wall thickness of the inner tube (10) to an inner diameter of the inner tube (10) ranging from 0.1 to 10; and
one braided layer or more braided layers (20) that are successively overlapped from inside to outside, the braided layers (20) being disposed on an outer periphery of the inner tube (10), each of the braided layers (20) comprising one flexible braided wire or more flexible braided wires (21) that are overlapped in an interlaced manner, and a wire diameter of the flexible braided wires (21) being 0.01-10mm.

2. The medical tubing according to claim 1, further comprising:
a cladding layer (30) disposed outer than the braided layers (20), wherein the cladding layer (30) is made of PVC, or polyurethane, or pp, or nylon, or rubber, or silica gel.

3. The medical tubing according to claim 1, wherein each of the braided layers (20) comprises at least two flexible braided wires (21) overlapped in an interlaced grid-like manner.

4. The medical tubing according to claim 3, wherein each of the flexible braided wires (21) and an axial direction of the inner tube (10) form an angle of 1°-89°.

5. The medical tubing according to claim 4, wherein each of the flexible braided wires (21) and the axial direction of the inner tube (10) form an angle of 40°-50°.

6. The medical tubing according to claim 5, wherein the flexible braided wires (21) include two groups, and the two groups of flexible braided wires (21) respectively extend towards two axially opposite ends of the inner tube (10).

7. The medical tubing according to claim 3, wherein the wire diameter of the flexible braided wires (21) is 0.5-1.5mm.

8. The medical tubing according to claim 3, wherein the ratio of the wall thickness of the inner tube (10) to the inner diameter of the inner tube (10) ranges from 0.5 to 2.

9. The medical tubing according to claim 8, wherein the wall thickness of the inner tube (10) is 0.5mm-1.5mm, and the inner diameter of the inner tube (10) is 1mm-1.5mm.

10. The medical tubing according to any one of claims 1-9, wherein the inner tube (10) is made of PVC, or polyurethane, or pp, or nylon, or rubber, or silica gel.

11. The medical tubing according to any one of claims 1-9, wherein the flexible braided wires (21) are made of stainless steel wire, or nylon wire, or KEVLAR^{®}.
